# EUROPEAN PATENT APPLICATION

(11) **EP 1 072 230 A1**
(43) Date of publication of application: **31.01.2001**
(21) Application number: 00306318.7
(22) Date of filing: 25.07.2000
(51) Int. Cl.: A61B 18/14

(54) **A bipolar ablation/coagulation electrode**

(30) Priority: 26.07.1999 US 145626 P; 02.03.2000 US 517411
(71) Applicant: ETHICON INC., Somerville New Jersey 08876 (US)
(72) Inventor: Tom, Curtis, Menlo Park, CA 94025 (US); Ciarrocca, Scott, Stockton, NJ 08559 (US)
(74) Representative: Mercer, Christopher Paul

(57) **Abstract**

The present invention provides devices, systems, and methods for using a single bipolar electrode structure for both resection and ablation. Generally, the electrode structure of the present invention comprises a wire loop used in conjunction with bipolar RF generators for use in a submerged saline environment. A supporting member of the wire loop defines at least one return electrode, which maintains a fixed distance from the active electrode.

## Description

### BACKGROUND OF THE INVENTION

### 1. Field of the Invention

This invention relates generally to electrosurgical devices, and in one particular aspect, provides a bipolar electrode for use in a conductive media that is adapted for both moving through and removing tissue, and for sliding over a tissue surface for ablation and / or coagulation.

### 2. Description of Background Art

Electrosurgery has been in use for many years as a surgical technique. For example, it is known to use electrosurgical cutting implements in transcervical fibroid removal to sever and remove uterine tissues. Such severing of tissues requires the use of high-power settings, typically an alternating current of between about 100 kHz and 2.2 GHz, and between about 500 and 16,000 volts. This energy is transmitted from a small cutting surface of a surgical implement (commonly referred to as the 'active' electrode) to the tissues at the surgical site. Uterine cavity distension can facilitate this electrosurgical procedure, typically with a nonconductive fluid such as Sorbitol or Mannitol or the like under sufficient pressure to separate the walls of the uterus and render the surgical site suitable for observation and access. The cutting surface of these implements usually consists of a wire, blade, ball, or other solid metallic shape, and the transmission of current to the tissue is predominantly monopolar, with the circuit being completed by a conductive pad (commonly referred to as the return electrode) applied to the patient's skin.

More recently, bipolar electrosurgery systems and devices have been proposed for use in conductive solutions such as saline, which is significantly safer than the non-physiologic distension media commonly employed. These bipolar devices incorporate a return electrode consisting of a relatively large conductive surface near the active electrode, so that the energy is sufficiently concentrated to affect tissue only at the active electrode adjacent the target tissues.

In both monopolar and bipolar systems, heat generated from the resistance of tissues to the concentrated flow of electrical current near the active electrode is high enough to vaporize cells. This allows a cut to be made with very little physical effort on the part of the surgeon and can provide a significant amount of tissue coagulation, which will result in good concomitant hemostasis.

Electrosurgical resistance heating may also be used at much lower power densities to denature and coagulate bleeding blood vessels and tissues. The active electrode portions of coagulation implements have greater surface areas compared to active electrodes designed specifically for cutting and are used to treat broad surfaces without physically penetrating the tissue. Such implements are used within the uterus to ablate the endometrial lining, and often take the form of a roller ball, roller barrel, or specialized fixed electrode to heat a wide swath of tissue with each stroke.

Electrosurgical implements equipped with small diameter wire loops are most commonly used for rapidly resecting tissue from a surgical site. These wire loops minimize total physician effort, particularly when severing relatively large amounts of tissues. Heat from the cutting element can also cauterize smaller blood vessels, preventing bleeding so that visibility at the operating site remains reasonably good.

While these differing electrosurgical methods and implements have been largely successful, they suffer from significant disadvantages. In particular, it is often desirable to use a cutting device for resection of fibroids and, during the same procedure, to cauterize any bleeding blood vessels or to ablate alternative portions of the uterus. Generally, this requires removal of the resection implement from the distended uterus and insertion of a dedicated vaporizing electrode. This device removal and replacement significantly increases cost and the time required for each procedure, and can complicate the fluid pressurization method and system to maintain distention throughout both procedures.

As a remedy to this problem, it has previously been proposed to use a single vaporizing electrosurgical implement for both tissue removal and cauterization. Unfortunately, the drag and relatively poor cutting performance inherent to known large ablation electrodes adds substantially to physician effort and procedure time during removal of tissues. In addition, high power settings associated with vaporization and severing of tissues often results in a build-up of hardened debris and untreated tissue on the active electrode, which further reduces device performance and visibility of the treatment site.

It has also been proposed to include two different electrode structures in a single implement. Unfortunately, this adds significantly to implement cost, complexity, and size, and may result in a less maneuverable device.

Finally, present bipolar implements are often constructed with a shaft consisting of two conductors or support structures arranged side-by-side to separate the active and return electrodes. This arrangement results in a relatively large surface area and irregular cross-section for the shaft. Hysteroscopes typically are equipped with a compliant seal to prevent fluid from leaking from the system; the side-by-side form of existing electrodes makes this seal difficult to maintain and greatly increases the drag on the electrode as it slides back and forth through the seal.

In light of the above, it would be advantageous to provide improved electrosurgical implements, systems, and methods for effecting tissue removal and hemostasis. It would be particularly advantageous to provide enhanced structures and techniques to facilitate resection, ablation, and coagulation with a single implement. Preferably, these improvements would minimize physician effort, avoid tissue adherence effects, minimize collateral tissue damage, and would be more efficient, but would not significantly increase complexity or cost over known electrosurgical systems.

Previous attempts at constructing bipolar loop electrosurgical implements involved using two wire loops (comprising the active and return electrodes) in close proximity to each other. Unfortunately, this configuration results in excessive drag across the tissue and produces uneven cutting effects. Further, previous attempts at constructing bipolar loops have included a mobile or transversing active loop and a stationary return loop. This configuration results in energy inefficiencies, mechanical complexity and uneven ablation / coagulation capabilities.

U.S. Patent No. 5,197,964 describes a bipolar instrument having a stationary electrode and a movable electrode. U.S. Patent No. 5,192,280 describes a pivoting multiple loop bipolar cutting device.

Thus, there is a need for an improved bipolar ablation / coagulation electrode that overcomes the deficiencies of the prior art.

### SUMMARY OF THE INVENTION

The present invention provides a bipolar electrocautery probe for use in a conductive media with electrosurgical instruments and systems, and methods for using a single electrocautery probe structure for resection, ablation, and coagulation that overcome the deficiencies of the prior art. Generally, the electrocautery probes of the present invention comprise fixed wire shapes to avoid the binding that often results when known rolling ablation / coagulation elements are powered with the high electrosurgical potentials used for removal of tissues. Electrosurgical current is concentrated on the small profile cutting surface of the wire and physician effort (drag) is avoided by minimizing the total profile of the device along the cutting axis.

In an exemplary embodiment, the probe comprises a single wire loop which is arranged at the distal end of an elongate shaft, and which constitutes the active electrode of the system. The wire loop is mounted between a pair of parallel arms, which, in part, form at least one return electrode. The return electrode, which is insulated from the active wire electrode, resides proximal to the active electrode on at least one of the arms and along the cutting axis to present a small cutting profile. This simple electrode structure provides a surface area that both engages and (when activated) passes through tissue and can be made to slide easily over tissue surfaces for ablation and coagulation.

In one aspect, the invention provides an electrosurgical electrocautery probe. The probe comprises a shaft having a proximal end, a distal end, and an axis therebetween. A formed wire is disposed at the distal end of the shaft and mounted between a mechanical support structure comprising a pair of arms. This wire defines an active electrode and is arranged so as to slide through or over a tissue surface when the probe is energized with an ablation or coagulation energy. A return electrode of substantially larger surface area than the active electrode is positioned proximally from the wire loop and is part of the mechanical structure supporting the wire loop. In this embodiment, the distance between the active electrode and return electrode is fixed, thus allowing constant and generally lower power requirements from the generator and eliminating the delivery of excess energy to the tissue. In this arrangement, the tissue will not reach the same temperatures as the prior art monopolar approaches. This results in highly localized tissue damage as a result of the minimal thermal damage to peripheral tissue. One clear benefit is that the tissue may be removed without excessive damage and therefore be more suitable for pathological evaluation.

In yet another aspect, the present invention provides an electrosurgical system comprising an electrosurgical potential source and an electrocautery probe. The potential source is capable of selectively supplying an ablation / coagulation energy and a tissue removal energy. The probe is couplable to the potential source, and includes both an active and return component which are aligned along a common axis. This alignment avoids drag when the potential source energizes the electrode with electrical energy, and the electrode is translated through tissue along the alignment axis. The electrode may also be slid over a tissue surface when the electrode is energized with a coagulation energy.

The present invention also provides an electrosurgical method. The method comprises energizing an electrocautery probe with a relatively high electrosurgical potential, and translating the highly energized structure through tissue and maintaining a fixed distance between the active electrode and return electrode. The electrosurgical structure can also be energized with a moderate electrosurgical potential and then moved along a surface of a tissue to raise the temperature of the tissue to effect ablation / coagulation. Hence, the method makes use of a single implement for both removal and coagulation of tissues.

These and other features and advantages of the present invention will become apparent from the following more detailed description, when taken in conjunction with the accompanying drawings which illustrate, by way of example, the principles of the invention.

### BRIEF DESCRIPTION OF THE DRAWINGS

FIGURE 1a is a perspective view of one embodiment of the electrocautery probe;
FIGURE 1b is an elevation view of the electrocautery probe of FIGURE 1;
FIGURE 1c is a plan view of the electrocautery probe Fig. 1;
FIGURE 2a is an enlarged elevation view of the distal end of the embodiment of Fig. 1a;
FIGURE 2b is an enlarged, partial cut-away plan view of the embodiment of Fig. 1;
FIGURE 2c is an enlarged view of the embodiment of Fig. 2b as seen along view line 2c-2c;
FIGURE 2d is an alternate embodiment of the loop electrode as seen along view line 2c-2c
FIGURE 3a is an enlarged, partial cut-away plan view of the distal end of an alternate embodiment of the electrocautery probe;
FIGURE 3b is an elevation view of the active electrode of the embodiment of Fig. 3a as seen along view line 3b-3b;
FIGURE 3c is an elevation view of the return electrode of the embodiment of Fig. 3a as seen along view line 3c-3c; and
FIGURE 4 is a diagram of a system for electrosurgically removing and ablating tissue using the electrocautery probe of the present invention and also shows a method for removal and subsequent coagulation or ablation of the endometrial lining of the uterus.

### DESCRIPTION OF THE PREFERRED EMBODIMENT OF THE INVENTION

Before explaining the present invention in detail, it should be noted that the invention is not limited in its application or use to the details of construction and arrangement of parts illustrated in the accompanying drawings and description, because the illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

The electrosurgical devices, systems, and methods of the present invention facilitate the removal, cauterization, and ablation of tissues during minimally invasive and open surgical procedures with a simple, fixed electrocautery probe. The present invention allows the physician to utilize a single electrode in conjunction with an endoscope, such as a resectoscope, for these different uses by varying the electrosurgical potential and the method of manipulating the probe. Thus, the physician may make use of the varying capabilities of the invention without removal and replacement of multiple devices at the surgical site, providing a significant advantage over known structures, particularly for minimally invasive surgical procedures which make use of a gaseous or liquid distension medium. Hence, these methods and devices will have advantageous applications in a wide variety of thoracic and urogenital procedures, including therapies and removal of tissues of the lung, tissues of the bladder, and tissues of the prostate. The most immediate application for the present invention will be for the removal, coagulation, and ablation of tissues within the uterus.

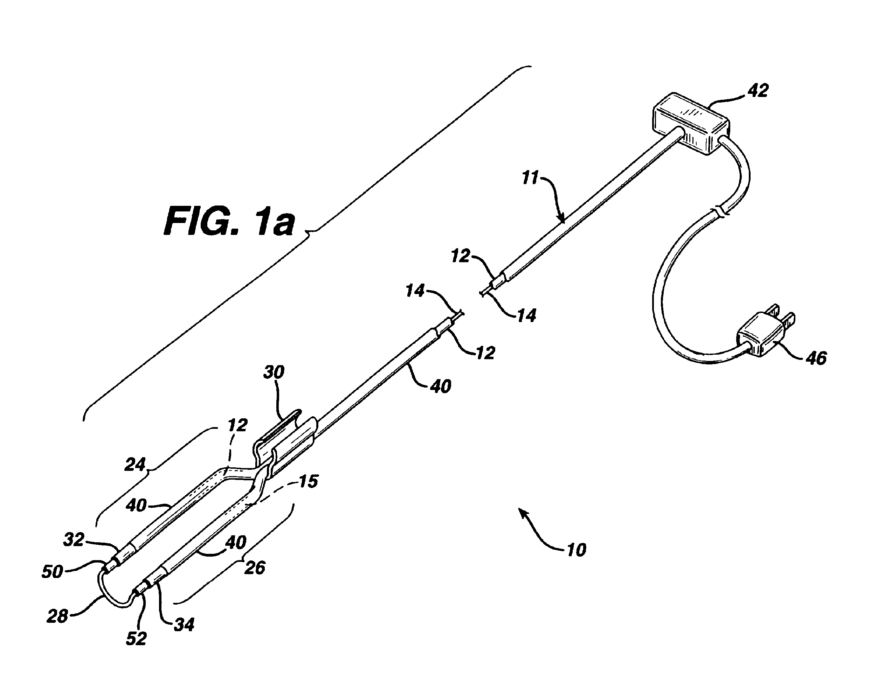

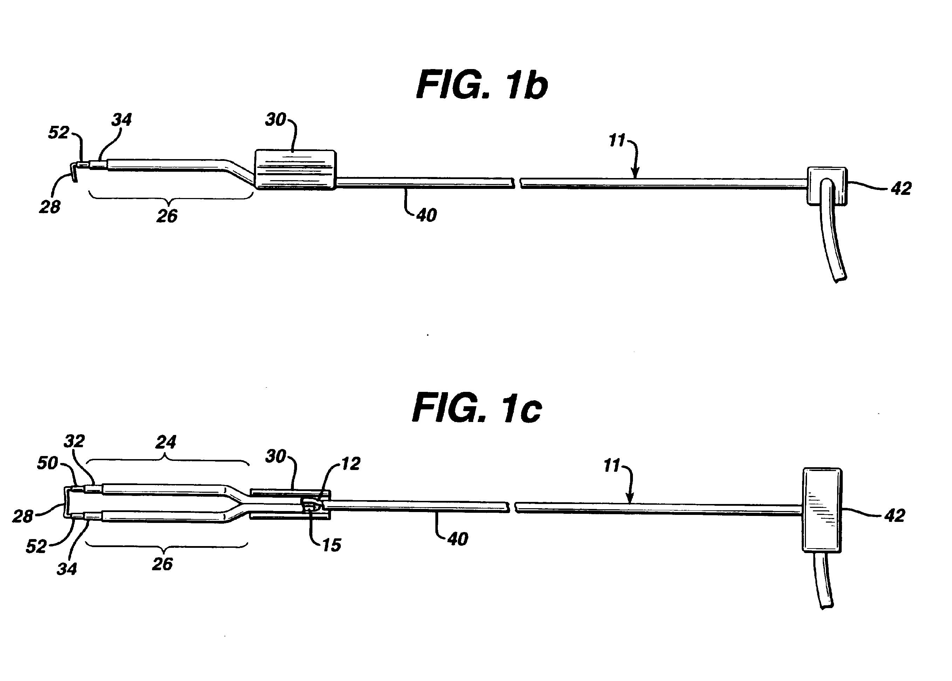

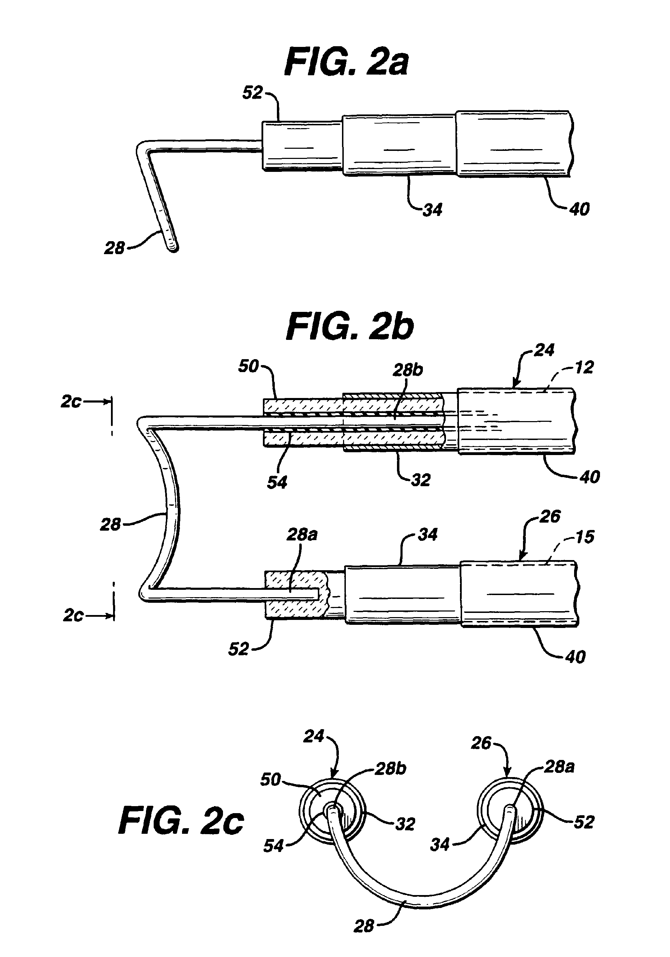

Referring now to Figs. 1a-c and 2a-d, a bipolar electrosurgical cautery probe 10 is defined in part by a shaft 11 having a proximal end and a distal end. Shaft 11 comprises an externally insulated electrically conducting tube 12 with an insulated electrically conducting wire 14 arranged coaxially within; tube 12 and wire 14 each having a proximal end and a distal end. Tube 12 is electrically insulated on its outer surface using an insulating material 40, such as PTFE, nylon or other suitable material. The proximal ends of tube 12 and wire 14 terminate within an electrical connector 42 and electrically couple via an electrical cable 44 and connector arrangement 46 to an electrosurgical power unit as is commonly known to those skilled in the art. At the distal end of the probe 10, at the general location of a resectoscope mounting sleeve 30, tube 15 attaches to tube 12, and tubes 12 and 15 diverge and form a pair of arms 24 and 26 which provide an optimal mechanical and functional arrangement for the active wire electrode 28 mounted between the arms 24 and 26. As is readily apparent to those skilled in the art, tube 15 may extend to the proximal end of shaft 11 to provide additional support to probe 10. Return electrodes 32 and 34 are formed by leaving all or a portion of arms 24 and 26 uninsulated and form a split and balanced arrangement that promotes uniform current density and tissue effect along the active electrode. Alternatively, the resectoscope shaft may be used as the return electrode by making the proper connection between the shaft and the electrosurgical power unit.

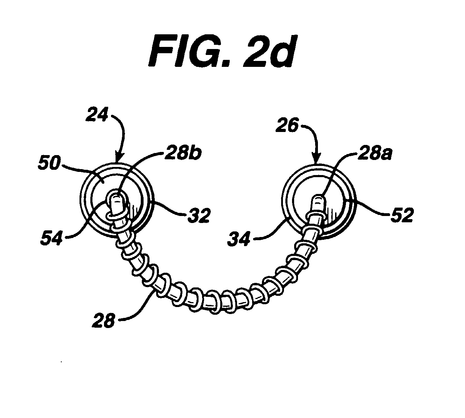

Referring specifically to Figs. 2a and b, hollow insulators 50 and 52 fit within the distal ends of tubes 12 and 15. Insulators 50 and 52 are preferably made of a high-temperature material, such as zirconia, aluminum-oxide, or glass. Active electrode 28 combines with insulators 50 and 52 as described below. Electrode 28 is preferably constructed from a high temperature metal or alloy such as tungsten, tungsten-rhenium, platinum-irridium, titanium, stainless steel, or similar biocompatible material. It may also be made from composite materials such as polymers with metal plating or metal loops with selectively masked areas for increased efficiency. This wire-form may also be comprised of a wire with a smaller wire tightly over-wrapping the entire length of or at least a portion of the conductor or a free-standing tightly wound coil as shown in Fig. 2d. Electrode end 28a terminates within insulator 52 as shown in Fig 2b and may be affixed therein using a high temperature epoxy or other mechanical means. Electrode end 28b extends through insulator 50 and through tube 12and electrically connects to insulated conductor 14. Alternately, electrode end 28b may pass through the entire length of tube 12 and terminates at electrical connector 42 and electrically couples as the active electrode via electrical cable 44 and plug 46 arrangement to an electrosurgical power unit as is commonly known to those skilled in the art. This alternate arrangement eliminates the necessity for conductor 14 and attachment of the active electrode wire thereto. Electrode end 28b is further insulated with an appropriate electrical insulator 54 for that portion that travels through tube 12 and all tubes proximal to prevent short circuits between the active and return electrode. This tubular design of the electrocautery probe provides a desirably small, low-resistance profile for passing through an elastomeric fluid seal.

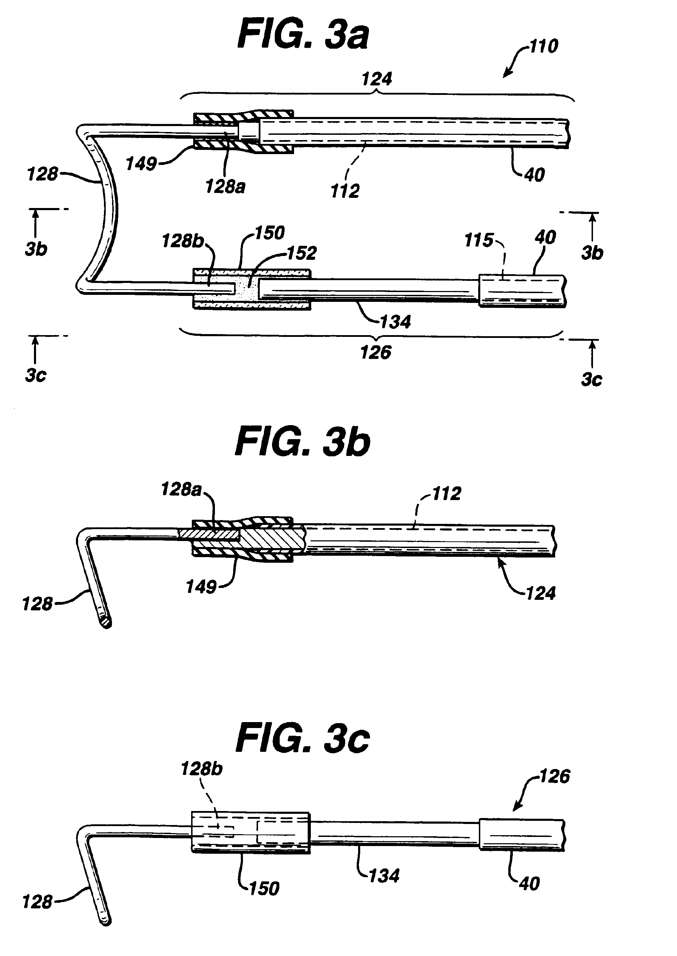

An alternate embodiment to the present invention is shown in Figs. 3a-c. For clarity purposes, Figs. 3a-c refer only to the distal end of probe ***110*** with the understanding that the overall probe construction resembles that as disclosed in Fig. 1a. A bipolar electrosurgical cautery probe 110 comprises two electrically conducting tubes 112 and 115, each having a proximal end and a distal end. At the distal end of the probe tubes 112 and 115 diverge and form a pair of arms 124 and 126 which provide an optimal mechanical and functional arrangement for the active wire electrode 128 mounted between the arms 124 and 126. Return electrode 134 is formed by leaving a portion of arm 126 uninsulated. The remaining surface of arm 126 may be insulated or uninsulated depending on the required current densities of a particular application. Arm 124 is completely insulated.

Formed between the arms 124 and 126 is the active electrode 128, preferably constructed from a high temperature metal or alloy such as tungsten, tungsten-rhenium, platinum-irridium, titanium, stainless steel, or similar biocompatible material. It may also be made from composite materials such as polymers with metal plating or metal loops with selectively masked areas for increased efficiency. One end 128a electrically connects via methods such as spot welding or soldering to tube 112. A heat shrink electrical insulating tube 149 preferably encloses end 128a and tubing 112 as shown. Alternatively, a separate conductor may travel through tube 112 and connect with end 128a and the electrical generator. End 128b of electrode 128 connects to one end of an insulator 150, preferably using a high temperature epoxy or some alternative mechanical means. Return electrode 134 terminates at the opposite end of insulator 150, also with a high temperature epoxy. Preferably, an insulating material 152 is disposed within insulator 150 between end 128b and electrode 134. This alternate electrosurgery cautery probe tubular design also provides a desirably small, low-resistance profile for passing through an elastomeric fluid seal.

When the electrocautery probe 10 is energized with an RF power source with a suitable waveform, it may be used to resect tissues by translating the shaft forward and backward through tissue or ablate tissues by translating the shaft forward and backward and engaging the active electrode 28 along a tissue surface. When the implement is energized with a lower power setting and an appropriate wave form and duty cycle, it may be used to coagulate tissue using the same motion.

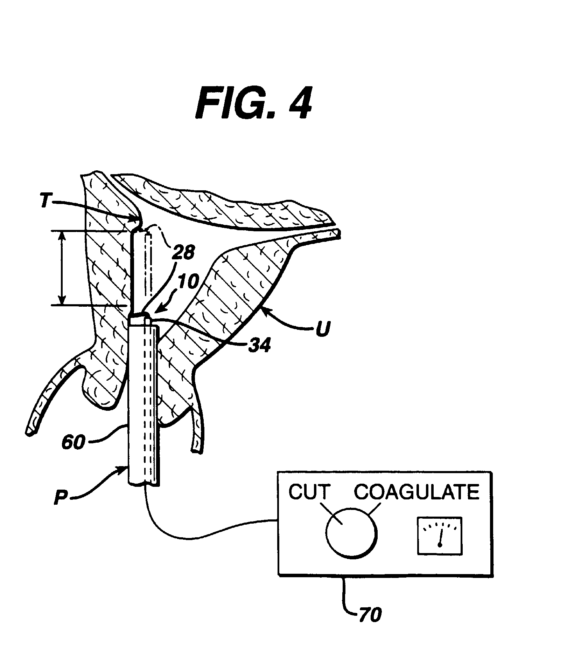

An exemplary system and method for using the implement of the present invention can be understood with reference to FIG. 4. In this illustration, a resectoscope or hysteroscope 60 of the prior art possessing inner and outer sheaths for fluid irrigation, a working channel, and an optical component for visualizing the electrode is disposed trans-cervically within a uterine cavity U. The cavity is distended via the irrigation sheaths of the resectoscope with a conductive fluid media such as normal saline, ringers lactate, or the like. The electrocautery probe 10 is deployed through the working channel of the resectoscope such that it can be brought into contact with intra-uterine tissue while in the field of view of the optical element of the resectoscope 60.

Probe 10 is energized using an electrosurgical power unit 70. Power unit 70 can selectably energize the probe 10 with a tissue removal electrosurgical energy or a tissue ablation electrosurgical energy. Generally, the probe 10 will be selectably energized with between about 50 to 150 Watts. For removal of tissues, probe 10 will typically be energized with an electrical potential providing a total of about 100 to 150 watts. When used for ablation and / or coagulation of tissue surfaces, power unit 70 will typically provide a total energy of about 50 watts to about 100 watts.

Tissue removal will be optically directed using the optical element of the resectoscope 60 while probe 10 is energized with a tissue removal potential. In some embodiments, the probe 10 will move axially independently from the sheath of the resectoscope 60, and the energized probe 10 will be drawn proximally through a tissue T toward a substantially fixed resectoscope 60 as shown in Fig. 4. In other embodiments, the resectoscope 60 and probe 10 will be drawn proximally together. After removal of a target tissue, the probe 10 can be used to coagulate any bleeding blood vessels and / or ablate tissues adjoining the newly formed tissue surface. Potential source 70 energizes the probe 10 with the more moderate ablation potential, and the probe 10 is then manipulated against the surface of the tissue.

In some embodiments, the impedance between the active and return electrodes is used in a feedback control system to vary the electrosurgical potential and control the depth and temperature of tissue heating.

In still another embodiment, a temperature sensing element such as a thermocouple, thermistor, resistance-thermal-device or the like will be arranged at the active electrode to provide a direct measure of tissue temperature. This temperature is used in a feedback control system to vary the electrosurgical potential and control the depth and temperature of tissue heating.

While the exemplary embodiments of the present invention have been described in some detail by way of illustration and for clarity, a wide variety of modifications, alternatives, and changes will be obvious to those who are skilled in the art. Therefore, the scope of the present invention is limited solely by the appended claims.

## Claims

1. A bipolar electrocautery probe having a first arm and a second arm between which an active electrode is mounted, the first arm comprising a return electrode, wherein the distance between the active electrode and return electrode remains fixed during use of the probe.

2. The bipolar electrocautery probe of claim 1 wherein the second arm comprises a return electrode and the distance between the active electrode and return electrode of the second arm remains fixed during use of the probe.

3. A bipolar electrode probe for use with a resectoscope comprising: a shaft having a proximal end and a distal end, the distal end terminating in a bifurcated support member supporting an active element and the bifurcated support member defining a return element at a fixed distance from the active element.

4. The probe of claim 3 wherein the bifurcated support member defines a second return element at a fixed distance from the active element.

5. An electrosurgical system for use with a high frequency power supply, the system comprising:
a) an electrosurgical probe comprising a shaft having a proximal end and a distal end, the distal end having a first arm and a second arm between which an active electrode is mounted and the first arm comprises a return electrode, wherein the distance between the active electrode and return electrode remains fixed during use of the probe, and a connector near the proximal end of the shaft for electrically connecting the active electrode and return electrode to the power supply wherein the electrosurgical probe is coupleable to an endoscopic instrument; and
b) an electrically conducting fluid supply connected to the endoscopic instrument and in electrical contact with and between the return electrode and active electrode for generating a current flow path between the return electrode and active electrode.

6. The electrosurgical system of claim 1 wherein the second arm comprises a second return electrode at a fixed distance from the active electrode.

7. The probe of any one of claims 1 to 4 or the system of claim 5 or claim 6, for use in applying electrical energy to a tissue target site.
